# EUROPEAN PATENT APPLICATION

(11) **EP 3 332 757 A1**
(43) Date of publication of application: **13.06.2018**
(21) Application number: 16833064.5
(22) Date of filing: 03.08.2016
(51) Int. Cl.: A61F 9/02, G02C 7/16

(54) **EYESHIELD AND MOUNTER**

(30) Priority: 04.08.2015 JP 2015154619
(71) Applicant: Ohura, Issei, Osaka-shi, Osaka 546-0033 (JP)
(72) Inventor: Ohura, Issei, Osaka-shi, Osaka 546-0033 (JP)
(74) Representative: Sharman, Thomas Alexander
(86) International application number: PCT/JP2016/072733
(87) International publication number: WO 2017/022785

(57) **Abstract**

Provided is a simple mounter that can, with the type of eye shield in which a sheet attaches to a frame, significantly reduce the time it takes to attach and remove a sheet to and from the frame and attach a sheet to the frame without soiling a surface of the sheet.

[Means to Achieve Object]

Two through-holes and a slit extending between the two through-holes are provided in each of bilaterally symmetric locations near a top edge of left and right sides of an eye shield sheet configured of an elastic and transparent resin sheet. The eye shield sheet is obtained by forming an elastic rod into an approximate U-shape securable to the head of a wearer, and includes a curved section at each temple region of the wearer that curves inward and is configured to fit into the slit and the two through-holes. Attachment of the sheet to the frame is completed by the wearer first bending the entire sheet and positioning the sheet on the inner side of the frame, and then inserting the curved sections of the frame one side at a time into the sheet simply by pressing the curved section against the slit to open the slit and continue pressing until the frame fits into the two through-holes.

## Description

### [Technical Field]

The present invention relates to a technology pertaining to an eye shield that is used by, for example, a healthcare practitioner and covers the upper part of the face.

### [Background Art]

Healthcare practitioners such as doctors and nurses wear masks that cover the lower part of the face when, for example, performing surgery, and wear eye shields that cover the upper part of the face above the mask when it is necessary to protect themselves from blood or body fluid splash from the patient.

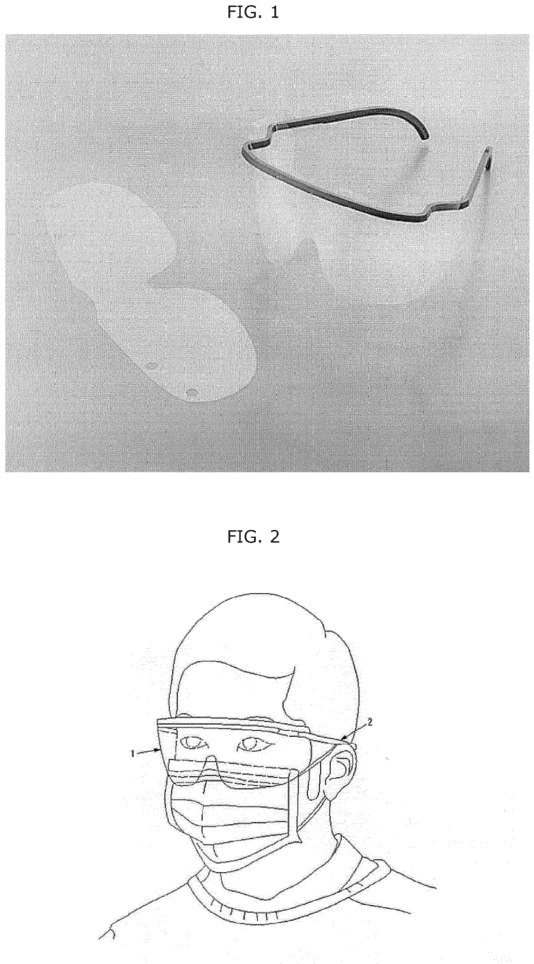

An eye shield including an elastic and transparent resin sheet that affixes to a mask by some means has been proposed, examples of which are disclosed by Patent Literatures 1 and 2. However, in recent years, use of eye shields that include a sheet that attaches to an elastic, approximately U-shaped frame made of, for example, resin, such as illustrated in FIG. 1, and are worn like glasses on the head and above a mask, such as illustrated in FIG. 2, has become widespread.
[Patent Literature 1] Japanese Unexamined Patent Application Publication No. H7-178117
[Patent Literature 2] Japanese Utility Model Registration No. 3160039

In medical settings, it is important for the healthcare practitioner to wear a mask and/or eye shield as a means for preventing themselves from becoming infected from the patient. Typically, a new mask and/or eye shield is used each time, and the mask and/or eye shield is disposed of after use. Regarding eye shields in particular, the sheet is typically a one-time use disposable sheet, and the frame is typically used repeatedly and disinfected after each use. However, in busy medical settings requiring urgent action, such as during surgery, the time it takes to attach and remove the sheet to and from the frame places extra strain on the healthcare practitioner, and fogging or soiling of the sheet reduces visibility. With the type of eye shield that attaches to a mask in particular, part of the sheet contacts the face. This is problematic not only because it applies stress to the wearer, but because it makes the eye shield prone to shifting out of place and becoming foggy or soiled.

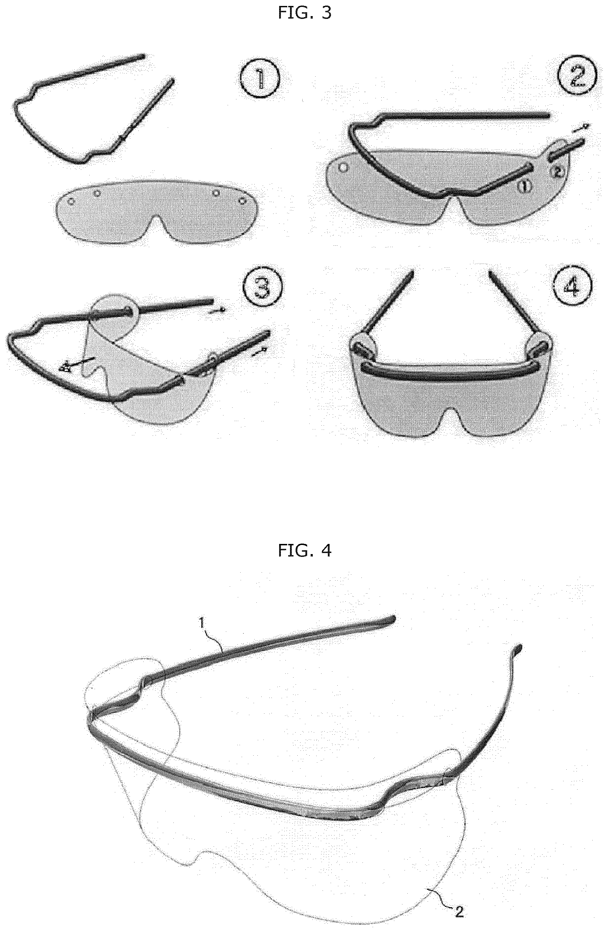

The type of eye shield in which a sheet attaches to a frame, such as the eye shield illustrated in FIG. 1, applies little stress to the wearer and is not prone to shifting out of place. However, when attaching the sheet to the frame, it is necessary to sequentially pass the ends of the frame through the two through-holes provided in each of the left and right sides of the sheet, as illustrated in FIG. 3. This is problematic in that the process is time consuming and prone to soiling the sheet.

### [Disclosure of Invention]

### [Problems that Invention is to Solve]

The present invention has been conceived to overcome the above-described problems with the conventional art, and in particular aims to provide a simple mounter that can, with the type of eye shield in which a sheet attaches to a frame, significantly reduce the time it takes to attach and remove a sheet to and from the frame and attach a sheet to the frame without soiling the surface of the sheet.

### [Means to Solve Problems]

In order to overcome the above-described problems, the present invention according to claim 1 is directed to an eye shield sheet configured of an elastic and transparent resin sheet and configured to removably attach as a single sheet to an eye shield frame and cover an upper part of a face of a wearer, the eye shield sheet including, in each of bilaterally symmetric locations near a top edge of left and right sides, two through-holes and a slit extending between the two through-holes.

Moreover, an eye shield according to claim 2 includes an eye shield frame (hereinafter simply referred to as "frame") and the eye shield sheet according to claim 1 (hereinafter simply referred to as "sheet"), the eye shield frame obtained by forming an elastic rod into an approximate U-shape securable to a head of a wearer and including, at each temple region of the wearer, a curved section that curves inward and is configured to fit into the slit and the two through-holes.

With this configuration, the method used to attach the sheet to the frame in the eye shield according to the present invention is fundamentally different from the attachment method used for the above-described conventional eye shield of the type in which a sheet attaches to a frame (hereinafter referred to as "conventional art"). With the conventional art, one end of the frame first needs to be inserted through the inner one the two through-holes from the front side of the sheet 2 and then inserted through the other outer one of the through-holes from the back side of the sheet 2 to insert the frame onto the sheet. The wearer needs to perform a total of three steps, namely, hold and bend the sheet with one hand so that the two through-holes face each other on the rear side of the sheet, insert an end of the frame through the two through-holes while holding the frame with one hand for both the left and right sides, and then move the sheet to the front of the frame. In time-critical situations, such as during surgery, this process not only places extra strain on the healthcare practitioner, but also increases the probability that the sheet will become soiled thereby reducing visibility as a result of the surface of the sheet being touched multiple times during the attachment process.

In contrast, with the configuration of the present invention, attachment is completed by the wearer first bending the entire sheet and positioning the sheet on the inner side of the frame, and then inserting the curved sections of the frame one side at a time into the sheet simply by pressing the curved section against the slit to open the slit and continue pressing until the frame fits into the two through-holes. Since it is not necessary to sequentially insert the ends of the frame through two through-holes and move the sheet to the front of the frame like is necessary with the conventional art, not only is the efficiency of the attachment process greatly improved, but the probability that the sheet will become soiled is reduced since it is possible to keep the locations that are touched with fingers to a minimum.

The invention according to claim 3 is directed to a mounter for attaching the eye shield sheet according to claim 1 to the eye shield frame, the mounter including: a mounting platform having a curved top surface corresponding to a shape of the eye shield sheet when attached, and including, on each of both side surfaces, a fitting hole corresponding to the two through-holes and the slit.

To attach the sheet, the wearer sets the sheet in a flat state on the mounting platform and then places the frame onto the mounting platform from above the sheet while holding both ends of the frame with both hands. Since the attaching of the sheet to the frame is completed by bringing the sheet pressed down by the frame into close contact with the mounting platform and pressing the curved sections of the frame so as to press open the slits and pass through the fitting holes, the frame just needs to be removed from the mounter in order to be able to put on the eye shield as-is.

This configuration is extremely efficient since the sheet can be attached to the frame in a single action after the sheet is set on the mounting platform. Moreover, since the wearer can attach the sheet to the frame without touching the surface of the sheet, the sheet remains substantially unsoiled.

Moreover, as described above, since these sheets are disposed of after one use in medical settings, the sheets are preferably stored in an easily reachable location in the area where the eye shield is to be used. If multiple sheets and frames are stocked on the mounter, the mounter can also be used as an eye shield dispenser, improving convenience.

### [Effects of Invention]

With the configuration according to the present invention, when a healthcare practitioner uses the eye shield, the healthcare practitioner can attach and remove the sheet to and from the frame efficiently and without soiling the surface of the sheet. This facilitates ubiquitous usage of eye shields in busy medical settings and contributes to the improvement of the safety of the healthcare practitioner and his or her medical service.

### [Best Mode for Carrying Out Invention]

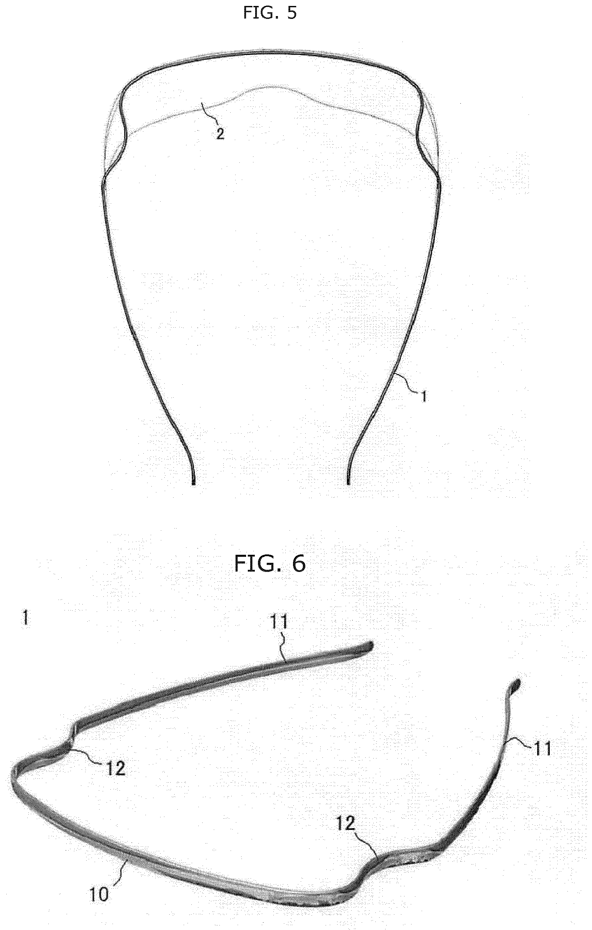

Hereinafter, an embodiment of the present invention will be described with reference to the drawings. FIG. 4 is a perspective view illustrating the appearance of an eye shield in the state it is to be used, i.e., with a sheet 2 attached to a frame 1, and FIG. 5 is a plan view of the same eye shield from above.

FIG. 6 is a perspective view of the frame 1. The frame 1 is an approximately U-shaped rod whose shape is defined by a front section 10 configured to be positioned in front of the head of the wearer and temple sections 11. The frame 1 also includes curved sections 12 configured to be positioned at both temples of the wearer that curve inward from the front section 10 to the temple sections 11. The material used for the frame 1 is not particularly limited so long as the material is elastic and flexible. For one-time use applications, a single piece of composite resin such as plastic is preferable from a cost aspect, and for repeated-use applications, composite resin with an antibacterial finish or a light metal such as aluminum or titanium alloy that can withstand being sterilized in a boiling water bath are preferable.

Note that with a conventional eye shield of the type in which a sheet attaches to a frame, the frame typically has a round cross section to save on manufacturing costs, but from the perspectives of improving contact with the head of the wearer, how the eye shield feels when worn, and the stability of the attached sheet, the cross section may be elliptical or approximately rectangular.

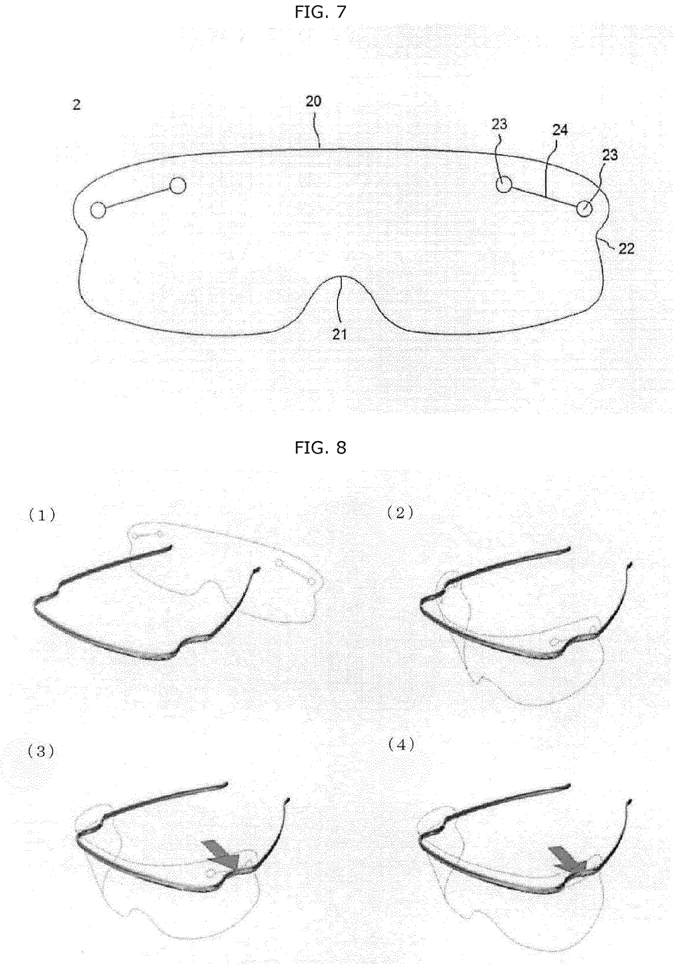

FIG. 7 is a plan view of the sheet 2 for use in the eye shield according to this embodiment. The sheet 2 is made of a single transparent resin sheet 20. The sheet 2 includes a bridge cut-out 21 in the lower central region that abuts the bridge of the nose. The sheet 2 also includes, near the top edge of each of the left and right sides, two through-holes 23 and a slits 24 between the two through-holes 23. The material used for the sheet 2 is not particularly limited so long as the material is a transparent resin that is elastic and flexible, but PET (polyethylene terephthalate) is preferable from a cost aspect. Note that the indentations 22 on both sides of the sheet 2 are provided to prevent interference between a glasses frame and the sheet 2 when the wearer of the eye shield also wears glasses (to be described later).

Note that since eye shields are often worn over masks and operating rooms are maintained at relatively high temperatures for medical reasons, the sheets often become foggy from the wearer exhaling or sweating. Accordingly, at least the surface of the sheet adjacent the face of the wearer is preferably treated with an anti-fog finish. Moreover, sheets that are, in principle, one-time use sheets are typically stocked in a stack of multiple sheets and as such are prone to surface scratches from rubbing together, but on the other hand, individually wrapping each sheet is costly. In view of this, during manufacturing of the sheet, it is preferable to apply a thin resin film to both sides while the sheet is still an uncut sheet of raw material and then stamp the raw sheets with a Thomson blade to produce the individual sheets. This way, the resin films can be removed immediately before the sheet is attached to the frame and the eye shield is used.

FIG. 8 is a sequence chart illustrating the process of attaching the sheet 2 to the frame 1 of the eye shield according to this embodiment. (1) At first, the sheet 2 is flat. (2) When attaching the sheet 2, the sheet 2 is bent and inserted so as to line the inner side of the frame 1 until the curved sections 12 of the frame 1 abut the slits 24 in the sheet 2. (3) A curved section 12 is pressed into a corresponding one of the left and right slits 24. (4) The curved section 12 penetrates through the slit 24 until both ends of the curved section 12 are fitted in the two through-holes 23 thereby affixing the sheet 2 to the frame 1. To complete the attachment of the sheet 2, the same steps are performed to affix the other side of the sheet 2 to the frame 1.

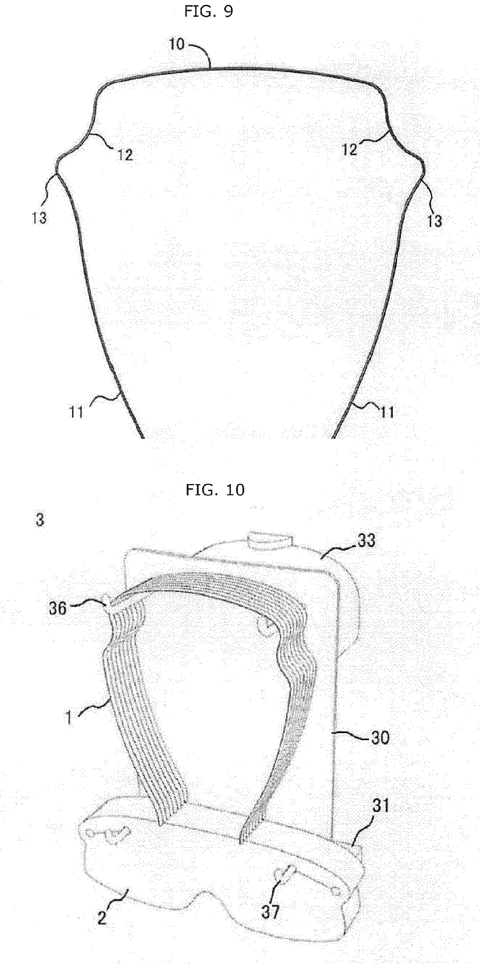

Since eye shields are also used by healthcare practitioners who regularly wear glasses, interference between the sheet 2 and the glasses is problematic. FIG. 9 is a plan view of one implementation example of the frame 1 that takes into consideration usage by wearers of glasses. By forming the left and right curved sections 12 to which the sheet 2 attaches so as to spread slightly outward, the degree to which the sheet 2 is bent can be minimized, and by forming second curved sections 13 that conversely curve outward on the temple sections 11 directly behind the curved sections 12, the surface of the sheet 2 adjacent to the face can be inhibited from interfering with the hinge regions of the glasses.

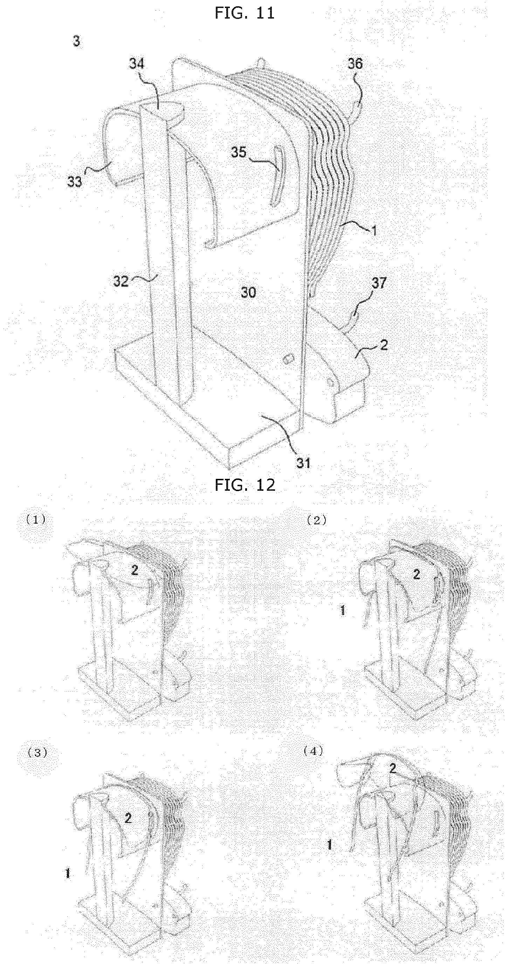

Next, FIG. 10 and subsequent drawings illustrate one implementation example of a mounter 3 for use with the eye shield according to the present embodiment. FIG. 10 and FIG. 11 are perspective views of the front and the back of the mounter 3, respectively.

The mounter 3 is designed to be used while placed on a tabletop. The mounter 3 includes a base 31, a plate 30 attached vertically to the base 31, a support pillar 32 attached vertically to the base 31 and disposed behind the plate 30, and a mounting platform 33 provided between the plate 30 and the support pillar 32. The mounting platform 33 has a curved top surface corresponding to the shape of the eye shield sheet when attached. The mounting platform 33 includes, on each of both side surfaces, a fitting hole 35 corresponding to the two through-holes 23 and the slit 24. Pairs of hooks 36 and pairs of hooks 37 are provided on the front side of the plate 30. The hooks 36 are for hanging the frames 1 and the hooks 37 are for hanging the sheets 2. A protrusion 34 having a shape corresponding to the bridge cut-out 21 of the sheet 2 is also provided on the rear of the top surface of the mounting platform 33.

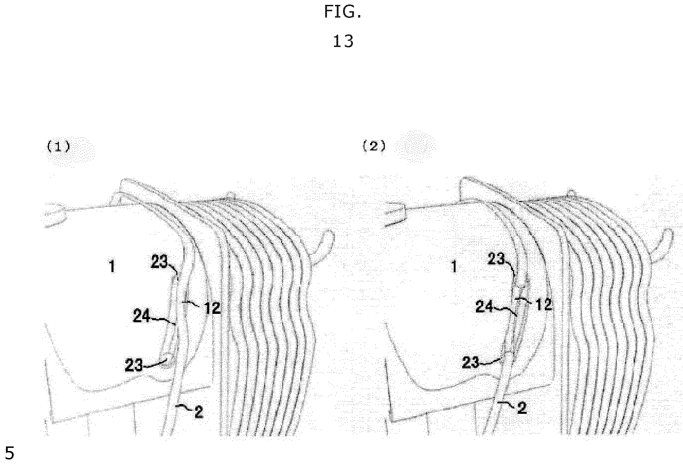

FIG. 12 is a sequence chart illustrating the process of attaching the sheet 2 to the frame 1 using the mounter 3, and FIG. 13 is an enlarged view illustrating the curved section 12 of the frame 1 being fitted into the slit 24 and the through-holes 23 of the sheet 2 in a fitting hole 35 of the mounting platform 33. (1) The wearer stands in front of the mounter 3, takes one sheet 2 off the hooks 37, and, with the top edge of the sheet 2 facing the wearer, places the sheet 2 onto the top surface of the mounting platform 33 while keeping the sheet 2 flat. Here, the sheet 2 is set in a predetermined position by fitting the bridge cut-out 21 against the protrusion 34. (2) Next, the wearer takes one frame 1 off the hooks 36, and places the frame 1 onto the mounting platform 33 from above the sheet 2 while spreading the terminal ends of the temple sections 11 using both hands. (3) In this state, the terminal ends of the frame 1 are brought back together in accordance with the elastic rebound of the temple sections 11, which brings the sheet 2 pressed down by the frame 1 into close contract with the curved surface of the mounting platform 33. Here, the curved sections 12 of the frame 1 press open the slits 24 of the sheet 2, penetrate through the fitting holes 35 in the mounting platform 33, whereby both ends of each curved section 12 fit into the two through-holes 23 to affix the sheet 2 to the frame 1. This completes the attaching. (4) Lastly, the wearer can remove the eye shield attached with the sheet 2 from the mounting platform 33 while holding the terminal ends of the temple sections 11 and place the eye shield on their head in a continuous movement. Note that the protective films (not shown in the drawings) on both sides of the sheet 2 are to be removed immediately before setting the sheet 2 in the predetermined position.

Usage of the mounter 3 makes it possible for the user to, after setting the sheet 2 on the mounting platform 33 and taking the frame 1 into their hand, attach the sheet 2 to the frame 1 with a single action without even touching the sheet 2 once, and put on the eye shield in a continuous movement. Accordingly, even in busy situations such as during surgery, a healthcare practitioner can rapidly put on the eye shield or switch out sheets 2 with minimal movement. Moreover, since there is a low probability of the sheet 2 becoming soiled, it is possible to maintain the visibility of the sheet 2 in top condition.

Hereinbefore specific configurations of a sheet and frame for use in an eye shield, an eye shield frame, and a mounter have been described, but the present invention is not limited to the embodiment described above. Improvements or changes are possible within the scope of the technical concept of the present invention, and such improvements and changes fall within the technical scope of the present invention.

### [Industrial Applicability]

The present invention is intended to be applied mainly to eye shields used for preventing healthcare practitioners from becoming infected in medical settings, but is also applicable to eye shields intended for various other uses in which there is a need to protect the eyes and/or face of the wearer from hazardous chemicals and/or fine particles. Moreover, by using a material that blocks ultraviolet rays on the sheet, the present invention is also applicable to simple sunscreen face guards for protecting the face from ultraviolet rays.

### [Brief Description of Drawings]

[FIG. 1] Example of a conventional eye shield.
[FIG. 2] Illustrates the sheet of a conventional eye shield when worn.
[FIG. 3] Illustrates an attachment method for an eye shield according to the present invention.
[FIG. 4] Perspective view of an eye shield according to an embodiment.
[FIG. 5] Plan view of an eye shield according to an embodiment.
[FIG. 6] Perspective view of a frame according to an embodiment.
[FIG. 7] Plan view of a sheet according to an embodiment.
[FIG. 8] Sequence chart illustrating the process of attaching a sheet according to an embodiment to a frame.
[FIG. 9] Plan view of a frame for use with glasses.
[FIG. 10] Front perspective view of a mounter according to an embodiment.
[FIG. 11] Rear perspective view of a mounter according to an embodiment.
[FIG. 12] Sequence chart illustrating the process of attaching a sheet to a frame using a mounter according to an embodiment.
[FIG. 13] Enlarged view illustrating the process of attaching a sheet to a frame using a mounter according to an embodiment.

### [Reference Signs List]

- 1: frame
- 10: front section
- 11: temple section
- 12: curved section
- 13: second curved section
- 2: sheet
- 20: transparent resin sheet
- 21: bridge cut-out
- 22: indentation
- 23: through-hole
- 24: slit
- 3: mounter
- 30: plate
- 31: base
- 32: support pillar
- 33: mounting platform
- 34: protrusion
- 35: fitting hole
- 36: hook (for holding the frames)
- 37: hook (for holding the sheets

## Claims

1. An eye shield sheet configured of an elastic and transparent resin sheet and configured to removably attach as a single sheet to an eye shield frame and cover an upper part of a face of a wearer, the eye shield sheet comprising, in each of bilaterally symmetric locations near a top edge of left and right sides, two through-holes and a slit extending between the two through-holes.

2. An eye shield comprising an eye shield frame and the eye shield sheet according to claim 1, the eye shield frame obtained by forming an elastic rod into an approximate U-shape securable to a head of a wearer and including, at each temple region of the wearer, a curved section that curves inward and is configured to fit into the slit and the two through-holes.

3. A mounter for attaching the eye shield sheet according to claim 1 to the eye shield frame, the mounter comprising: a mounting platform having a curved top surface corresponding to a shape of the eye shield sheet when attached, and including, on each of both side surfaces, a fitting hole corresponding to the two through-holes and the slit.
